# EUROPÄISCHE PATENTANMELDUNG

(11) **EP 2 664 304 A2**
(43) Veröffentlichungstag der Anmeldung: **20.11.2013**
(21) Anmeldenummer: 13002518.2
(22) Anmeldetag: 14.05.2013
(51) Int. Cl.: A61F 2/64

(54) **Prothesengelenk mit Sperrvorrichtung**

(30) Priorität: 14.05.2012 DE 102012009653
(71) Anmelder: Streifeneder ortho.production GmbH, 82275 Emmering (DE)
(72) Erfinder: Krafczyk, Thomas, 82140 Olching (DE); Schmid, Johannes, 80339 München (DE); Seitz, Verena, 82223 Eichenau (DE)
(74) Vertreter: Bosch, Matthias

(57) **Zusammenfassung**

Die vorliegende Erfindung bezieht sich auf ein Prothesengelenk (1) mit Sperrvorrichtung. Das Prothesengelenk (1) umfasst ein Oberteil (2), ein Unterteil (3) und ein Sperrelement (4), wobei das Oberteil (2) und das Unterteil (3) so angeordnet sind, dass das Prothesengelenk (1) durch Rotieren des Oberteils (2) gegenüber dem Unterteil (3) beugbar ist. Das Sperrelement (4) ist zudem so zwischen einer Freigabestellung (40) und einer Sperrstellung (30) überführbar ausgebildet ist, dass in der Sperrstellung (30) das Prothesengelenk (1) in einem vorgegebenen Beugungszustand fixierbar ist durch Verspannung des Sperrelements (4) zwischen dem Oberteil (2) und dem Unterteil (3), und eine Fixierung des Prothesengelenks (1) durch Überführen des Sperrelements (4) in die Freigabestellung (40) aufhebbar ist. Vorzugsweise umfasst das Prothesengelenk (1) mit Sperrvorrichtung außerdem einen Sperrhebel (5), der so angeordnet und ausgebildet ist, dass das Sperrelement (4) durch Verlagern des Sperrhebels (5) zwischen der Freigabestellung (40) und der Sperrstellung (30) überführbar ist.

## Beschreibung

Gegenstand der vorliegenden Erfindung ist ein Prothesengelenk mit einer Sperrvorrichtung.

Die Sperrvorrichtung dient dazu, das Prothesengelenk in einem bestimmten Beugungszustand zu fixieren. Während das Prothesengelenk also grundsätzlich beliebig gebeugt oder gestreckt werden kann, wodurch sich der Winkel zwischen den beiden Elementen, die durch das Prothesengelenk miteinander verbunden werden, ändert, kann das Prothesengelenk, sobald es in einem bestimmten Beugungszustand fixiert ist, weder gebeugt, noch gestreckt werden. Das erfindungsgemäße Prothesengelenk mit Sperrvorrichtung ist daher für Amputierte mit geringen Mobilitätsgraden geeignet, die ein sehr sicheres Gelenk benötigen, da das Prothesengelenk über die Sperrvorrichtung steif gemacht werden kann. Das erfindungsgemäße Prothesengelenk mit Sperrvorrichtung kann beispielsweise als Kniegelenk ausgebildet sein. In eine Oberschenkelprothese integriert, dient das Prothesengelenk dann dazu, ein Beugen des Unterschenkels gegenüber dem Oberschenkel zu ermöglichen.

Aus dem Stand der Technik sind Oberschenkelprothesen mit einem künstlichen Kniegelenk bekannt. Die WO 2005/112837 A1 beschreibt ein derartiges Kniegelenk. Dieses Kniegelenk weist lösbare Feststellmittel auf, über die es in einer bestimmten Beugestellung fixierbar ist. Die in WO 2005/112837 A1 beschriebene Lösung hat jedoch den Nachteil, dass das Kniegelenk selbst dann, wenn es fixiert ist, nicht vollständig steif ist. Dies ist dem Aufbau des Kniegelenks geschuldet. Das Kniegelenk weist nämlich ein Knieoberteil, ein Knieunterteil und eine diese verbindende Klemmfaust auf, wobei das Knieoberteil um eine Drehachse der Klemmfaust drehbar angeordnet ist. Die lösbaren Feststellmittel für die Fixierung mindestens einer Drehposition sind zwischen dem Knieoberteil und der Klemmfaust angeordnet. Bei dem in der WO 2005/112837 A1 beschriebenen Ausführungsbeispiel des Kniegelenks handelt es sich bei dem lösbaren Feststellmittel um eine Klinke, die schwenkbar um einen Bolzen über der Klemmfaust an dem Knieoberteil gelagert ist. Die Klinke kann in der Extensionsstellung des Kniegelenks in eine Ausnehmung der Klemmfaust eingerückt werden und sperrt so die Bewegung des Knieoberteils. Dadurch ist jedoch nur die Bewegung des Knieoberteils gegenüber der Klemmfaust gesperrt. Die Bewegung der Klemmfaust gegenüber dem Knieunterteil ist hingegen nicht gesperrt. Vielmehr kann sich die Klemmfaust nach wie vor um die Bremsanlenkachse gegenüber dem Knieunterteil drehen. Dies hat zur Folge, dass das Knie, wenn es vom Benutzer belastet wird, selbst dann um einen Winkel von wenigen Grad einknicken kann, wenn es fixiert ist. Besonders bei unerfahrenen Prothesenbenutzern, wie zum Beispiel frisch Amputierten, kann dies zu Verunsicherung führen.

Es ist daher die Aufgabe der vorliegenden Erfindung, ein Prothesengelenk mit Sperrvorrichtung anzugeben, bei dem sichergestellt ist, dass es in gesperrtem Zustand vollständig fixiert ist.

Diese Aufgabe wird durch das Prothesengelenk mit Sperrvorrichtung mit den Merkmalen gemäß Anspruch 1 gelöst. Vorteilhafte Ausgestaltungen der vorliegenden Erfindung sind in den Unteransprüchen 2 bis 10 angegeben.

Das erfindungsgemäße Prothesengelenk mit Sperrvorrichtung umfasst ein Oberteil, ein Unterteil und ein Sperrelement, wobei das Oberteil und das Unterteil so angeordnet sind, dass das Prothesengelenk durch Rotieren des Oberteils gegenüber dem Unterteil beugbar ist. Das Sperrelement ist so zwischen einer Freigabestellung und einer Sperrstellung überführbar ausgebildet, dass in der Sperrstellung das Prothesengelenk in einem vorgegebenen Beugungszustand fixierbar ist durch Verspannung des Sperrelements zwischen dem Oberteil und dem Unterteil, und eine Fixierung des Prothesengelenks durch Überführen des Sperrelements in die Freigabestellung aufhebbar ist. Dadurch, dass das Sperrelement in der Sperrstellung zwischen dem Oberteil und dem Unterteil verspannt werden kann, kann sichergestellt werden, dass das Prothesengelenk in gesperrtem Zustand vollständig fixiert ist. Dabei ist es vorzugsweise in der vollständig gestreckten Stellung fixiert. Dann ist das erfindungsgemäße Prothesengelenk, wenn sich das Sperrelement in der Sperrstellung befindet, im gestreckten Zustand durch Verspannung des Sperrelements zwischen dem Oberteil und dem Unterteil fixiert, so dass es nicht gebeugt werden kann. Befindet sich das Sperrelement hingegen in der Freigabestellung, verhindert die Sperrvorrichtung das Beugen des Prothesengelenks nicht. Erfindungsgemäß ist es auch möglich, das Prothesengelenk so auszubilden, dass es in einer anderen als der vollständig gestreckten Stellung, insbesondere auch in mehreren verschiedenen Stellungen, fixierbar ist.

Vorteilhafterweise umfasst das erfindungsgemäße Prothesengelenk mit Sperrvorrichtung weiterhin einen Sperrhebel, der so angeordnet und ausgebildet ist, dass das Sperrelement durch Verlagern des Sperrhebels zwischen der Freigabestellung und der Sperrstellung überführbar ist. Der Sperrhebel ist zweckmäßigerweise so ausgebildet, dass das Verlagern des Sperrhebels zum Überführen des Sperrelements aus der Sperrstellung in die Freigabestellung nur einen geringen Krauftaufwand erfordert. Wenn der Sperrhebel und das Sperrelement als zwei separate Bauelemente ausgeführt sind, lässt sich eine leichte Montierbarkeit des Prothesengelenks realisieren. Erfindungsgemäß ist es jedoch auch möglich, Sperrhebel und Sperrelement einteilig auszubilden. Beispielsweise kann das Sperrelement als zapfenförmiger Bereich des Sperrhebels ausgebildet sein.

Vorteilhafterweise umfasst das erfindungsgemäße Prothesengelenk mit Sperrvorrichtung weiterhin ein Betätigungsmittel, über das der Sperrhebel verlagerbar ist. Dieses Betätigungsmittel ist vorzugsweise so angeordnet, dass es für den Benutzer des Prothesengelenks leicht zugänglich ist. Beispielsweise könnte das Betätigungsmittel als Zugseil ausgebildet sein.

Das Sperrelement ist vorzugsweise als Sperrbolzen ausgebildet. Der Sperrhebel ist dann zweckmäßigerweise so ausgebildet und angeordnet, dass er den Sperrbolzen zumindest teilweise umgreift, so dass der Sperrbolzen durch Betätigen des Sperrhebels sowohl aus der Sperrstellung in die Freigabestellung verschiebbar ist, als auch in umgekehrter Richtung.

Weiterhin ist es vorteilhaft, wenn das Sperrelement im Oberteil gelagert ist. Das Oberteil weist dann vorzugsweise eine Aussparung auf, in der das Sperrelement verschiebbar gelagert ist. Wenn das Sperrelement im Oberteil angeordnet ist, lässt es sich in einfacher Weise so positionieren, dass es für den Benutzer leicht zugänglich ist, so dass er es leicht von der Sperrstellung in die Freigabestellung überführen kann.

Das erfindungsgemäße Prothesengelenk mit Sperrvorrichtung ist zudem vorzugsweise so ausgebildet, dass das Sperrelement in der Sperrstellung an einer Anlagefläche des Oberteils und an einer Anlagefläche des Unterteils anliegt, wenn das Prothesengelenk fixiert ist, und dass die Anlagefläche des Oberteils zu der Anlagefläche des Unterteils in einem Winkel von weniger als 15 Grad, vorzugsweise zwischen 3 Grad und 8 Grad, besonders bevorzugt von etwa 5 Grad, angeordnet ist. Dadurch, dass die Anlageflächen des Oberteils und des Unterteils nicht parallel zueinander angeordnet sind, sondern leicht geneigt zueinander, wird ein Verspannen des Sperrelements zwischen dem Oberteil und dem Unterteil erleichtert. Zwischen den leicht zueinander geneigten Anlageflächen lässt sich das Sperrelement nämlich in einfacher Weise spielfrei klemmen.

Das erfindungsgemäße Prothesengelenk mit Sperrvorrichtung umfasst vorzugsweise weiterhin ein elastisches Element, das eine Kraft erzeugt, die bestrebt ist, das Sperrelement in die Sperrstellung zu überführen. Diese Kraft sorgt dafür, dass das Sperrelement selbstständig aus der Freigabestellung in die Sperrstellung strebt. Dem Benutzer wird es somit abgenommen, das Sperrelement von der Freigabestellung in die Sperrstellung zu überführen. Das elastische Element sorgt zudem für zusätzliche Sicherheit, da auf diese Weise dafür gesorgt werden kann, dass sich das Sperrelement vorzugsweise im Sperrzustand befindet. Das elastische Element ist vorteilhafterweise als Feder, beispielsweise als Doppelschenkelfeder, ausgebildet.

Vorteilhafterweise umfasst das erfindungsgemäße Prothesengelenk mit Sperrvorrichtung weiterhin eine Bremsschelle, die so zwischen dem Oberteil und dem Unterteil angeordnet ist, dass die Bremsschelle gegenüber dem Oberteil um eine Bremsachse drehbar ist und gegenüber dem Unterteil um eine Steuerachse drehbar ist. Das erfindungsgemäße Prothesengelenk ist dann vorzugsweise so ausgebildet, dass in der Sperrstellung des Sperrelements die Drehbarkeit des Oberteils gegenüber der Bremsschelle um die Bremsachse sperrbar ist. Das erfindungsgemäße Prothesengelenk kann auch so ausgebildet sein, dass in der Sperrstellung des Sperrelements die Drehbarkeit der Bremsschelle gegenüber dem Unterteil um die Steuerachse sperrbar ist. Wenn in der Sperrstellung des Sperrelements die Drehbarkeit sowohl um die Bremsachse, als auch um die Steuerachse sperrbar ist, lässt sich ein Prothesengelenk realisieren, das in einer bestimmten Stellung vollständig fixiert werden kann. Dieses Prothesengelenk weist dann, anders als das aus der WO 2005/112837 A1 bekannte Prothesengelenk, im fixierten Zustand kein Bremsspiel auf.

Nachfolgend wird eine bevorzugte Ausführungsform der vorliegenden Erfindung anhand von Figuren näher beschrieben.

Es zeigen:
Figur 1 eine Schnittdarstellung des erfindungsgemäßen Prothesengelenks mit Sperrvorrichtung;
Figur 2A eine perspektivische Detailansicht des Prothesengelenks, wobei sich das Sperrelement in der Freigabestellung befindet und das Prothesengelenk gestreckt ist;
Figur 2B eine weitere perspektivische Detailansicht, wobei sich das Sperrelement wieder in der Freigabestellung befindet und das Gelenk gebeugt ist;
Figur 3 eine Detailansicht des Sperrhebels mit dem Sperrelement und von Ausschnitten des Oberteils sowie des Unterteils;
Figur 4 eine Detailansicht des Sperrelements und der Anlageflächen des Oberteils sowie des Unterteils, an denen das Sperrelement in der Sperrstellung anliegt.

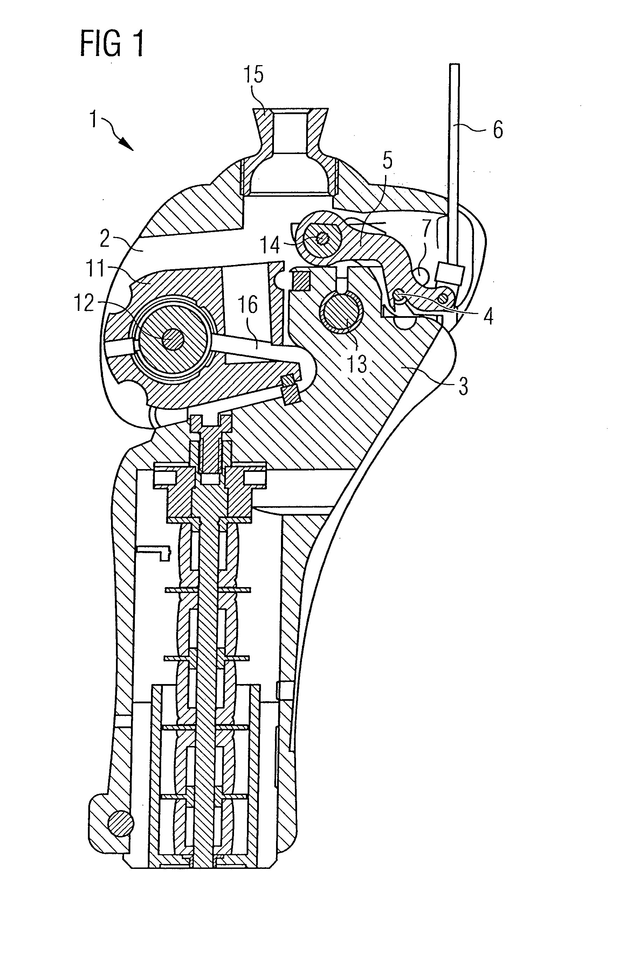

Figur 1 zeigt eine Schnittdarstellung des erfindungsgemäßen Prothesengelenks 1 mit Sperrvorrichtung. Das Prothesengelenk 1 umfasst ein Oberteil 2, ein Unterteil 3 und ein Sperrelement 4. Um eine standardisierte proximale Anschlussmöglichkeit zu bieten, ist am Oberteil 2 eine Pyramide 15 vorgesehen. Das Oberteil 2 weist zudem eine Aussparung 7 auf, in der das Sperrelement 4, das in Form eines Sperrbolzens ausgebildet ist, verschiebbar gelagert ist. Der Sperrhebel 5 ist um eine Drehachse 14 schwenkbar am Oberteil 2 gelagert und umgreift das Sperrelement 4. An dem Sperrhebel 5 ist ein Betätigungsmittel 6 in Form eines Zugseils angeordnet. Das Prothesengelenk gemäß Figur 1 weist zudem eine Bremsschelle 11 auf, die über eine Bremsachse 12 mit dem Oberteil 2 und über eine Steuerachse 13 mit dem Unterteil 3 verbunden ist.

Wird in dem in Figur 1 dargestellten gestreckten Zustand des Prothesengelenks 1 von oben über die Pyramide 15 eine Kraft auf das Prothesengelenk 1 ausgeübt, wird ein Drehmoment erzeugt, das versucht, das Oberteil 2 zusammen mit der Bremsschelle 11 entgegen dem Uhrzeigersinn um die Steuerachse 13 zu drehen. Durch eine derartige Drehung wird der Spalt 16 in der Bremsschelle 11 verengt, da die Bremsschelle 11 auf dem Unterteil 3 aufliegt, so dass sich die Bremsschelle 11 um die Bremsachse 12 festzieht. Diese Drehung von Oberteil 2 und Bremsschelle 11 um die Steuerachse 13, die man auch als Bremsspiel bezeichnen kann, wird bei dem erfindungsgemäßen Prothesengelenk 1 gemäß Figur 1 durch die Sperrvorrichtung verhindert, falls sich das Sperrelement 4, wie gezeigt, in der Sperrstellung befindet. Dann ist das Sperrelement 4 nämlich so zwischen dem Oberteil 4 und dem Unterteil 3 verspannt, dass sich das Oberteil 2 nicht entgegen dem Uhrzeigersinn um die Steuerachse 13 gegenüber dem Unterteil 3 drehen kann. Da die Sperrvorrichtung auch eine Drehung des Oberteils 2 um die Bremsachse 12 sperrt, ist das Prothesengelenk 1 vollständig steif und in der gestreckten Stellung fixiert.

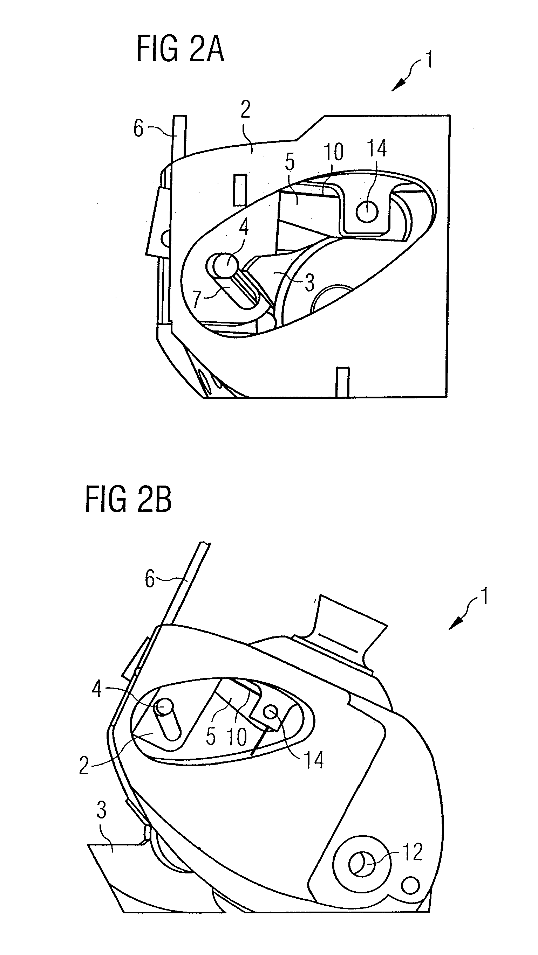

Figur 2A zeigt einen Ausschnitt aus dem erfindungsgemäßen Prothesengelenk mit Sperrvorrichtung im gestreckten Zustand. Durch Ziehen an dem Zugseil 6 wurde das Sperrelement 4 über den Sperrhebel 5 gegen die Kraft der Feder 10 in die Freigabestellung gebracht, indem es in der Aussparung 7 nach oben verschoben wurde. Wie in Figur 2A ersichtlich, liegt das Sperrelement 4 in der Freigabestellung nicht an der nasenförmigen Ausbuchtung des Unterteils 3 an, die an die Aussparung 7 angrenzend angeordnet ist. Die Sperrvorrichtung ist also entriegelt. Der Zustand des Prothesengelenks in Figur 2B unterscheidet sich von dem in Figur 2A dargestellten Zustand dadurch, dass das Prothesengelenk nunmehr gebeugt wurde. Das Beugen des Prothesengelenks 1 wird realisiert durch Rotieren des Oberteils 2 um die Bremsachse 12 gegenüber dem Unterteil 3. Das Sperrelement 4 befindet sich nach wie vor in der Freigabestellung, da der Zug auf das Zugseil 6 aufrechterhalten wurde und die Feder 10 den Sperrhebel 5 somit nicht nach unten drücken kann. Wenn das Zugseil 6 in dieser Stellung jedoch losgelassen wird, drückt die Feder 10 den Sperrhebel 5 nach unten, wodurch das Sperrelement 4 aus der Freigabestellung in die Sperrstellung überführt wird. Wird dann das Prothesengelenk wieder gestreckt, läuft das Sperrelement 4 auf den Rücken der nasenförmigen Ausbuchtung des Unterteils 3 auf. Der Rücken dieser Ausbuchtung, die in Figur 2A gut sichtbar ist, ist so geformt, dass das Sperrelement 4 im weiteren Verlauf der Beugebewegung durch die nasenförmige Ausbuchtung des Unterteils 3 aus der Sperrstellung in der Aussparung 7 so weit nach oben in Richtung Freigabestellung geschoben wird, dass das Sperrelement 4 die nasenförmige Ausbuchtung des Unterteils 3 passieren kann. Sobald das Prothesengelenk gestreckt ist, wird das Sperrelement 4 dann durch die Kraft der Feder 10 vom Sperrhebel 5 wieder in der Aussparung 7 nach unten in die Sperrstellung geschoben. Das Prothesengelenk ist dann wieder in der gestreckten Stellung fixiert.

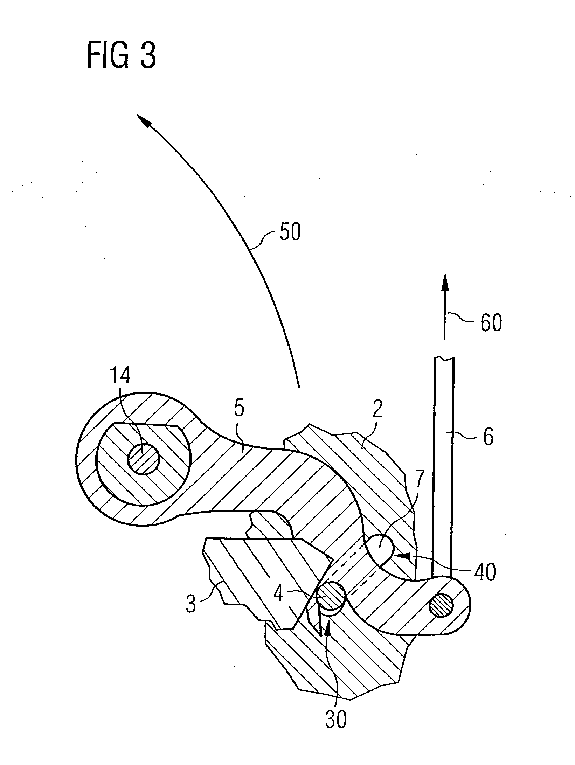

Figur 3 zeigt eine Detailansicht des Sperrhebels 5, der so ausgebildet ist, dass er das Sperrelement 4, das als Sperrbolzen ausgebildet ist, umfasst. Das Sperrelement 4 wiederum ist in einer langlochförmigen Aussparung 7 angeordnet, die in dem Oberteil 2 vorgesehen ist. Der Sperrhebel 5 ist um die Drehachse 14 drehbar am Oberteil 2 gelagert und weist an seinem der Drehachse 14 gegenüberliegenden Ende das Zugseil 6 auf. Ferner ist in Figur 3 der Teil des Unterteils 3 dargestellt (nasenförmige Ausbuchtung), der zusammen mit einem Bereich des Oberteils 2 für die Verspannung des Sperrelements 4 in der Sperrstellung führt. Um das Prothesengelenk zu beugen, müsste das Oberteil 2 gegenüber dem Unterteil 3 in Richtung des Pfeils 50 verlagert werden. Dies verhindert das Sperrelement 4, das in der Sperrstellung 30 zwischen das Unterteil 3 und das Oberteil 2 gespannt bzw. geklemmt ist. Um das Prothesengelenk beugen zu können, muss das Sperrelement 4 zuerst aus der Sperrstellung 30 in die Freigabestellung 40 überführt werden. Dazu zieht der Benutzer in Richtung des Pfeils 60 an dem Zugseil 6, so dass sich der Sperrhebel 5 entgegen dem Uhrzeigersinn um seine Drehachse 14 dreht und dabei das Sperrelement 4 in der Aussparung 7 nach oben verschiebt. Wenn sich das Sperrelement 4 in der Freigabestellung 40 befindet, ist es nicht mehr zwischen Oberteil 2 und Unterteil 3 verspannt, so dass das Prothesengelenk dann gebeugt werden kann.

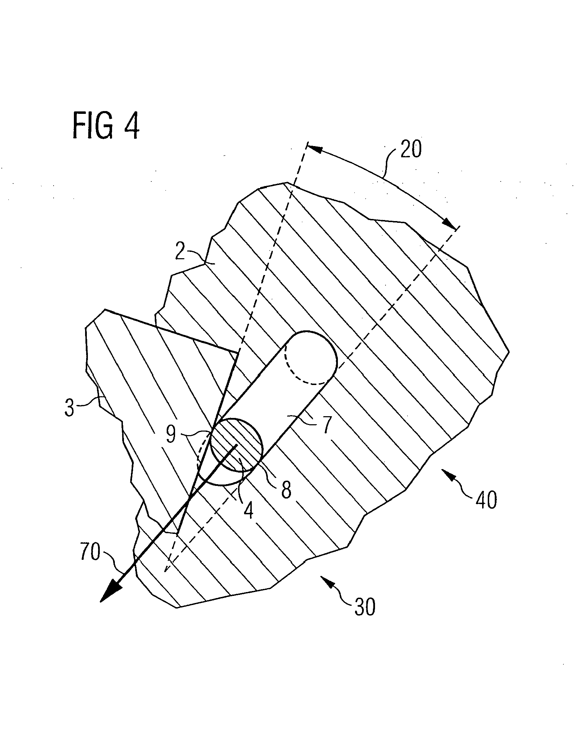

In Figur 4 ist der Teil des Oberteils 2 dargestellt, in dem die Aussparung 7 vorgesehen ist, in der das Sperrelement 4 angeordnet ist. Die untere Kante der Aussparung 7 bildet die Anlagefläche 8 des Oberteils 2, an der das Sperrelement 4 im Sperrzustand anliegt. Weiterhin ist in Figur 4 ein Teil des Unterteils 3 dargestellt. Die Anlagefläche 9 des Unterteils 3, an der das Sperrelement im Sperrzustand 30 ebenfalls anliegt, wird durch einen Kantenbereich des Unterteils 3 gebildet. Die Anlagefläche 8 des Oberteils 2 und die Anlagefläche 9 des Unterteils 3 sind leicht geneigt zueinander angeordnet und schließen den Winkel 20 zwischen sich ein. Um den Effekt der leicht zueinander geneigt angeordneten Anlageflächen 8 und 9 besser veranschaulichen zu können, ist in den Figuren 3 und 4 der Winkel 20 zwischen den Anlageflächen 8 und 9 bewusst groß dargestellt.

Durch das elastische Element 10 (nicht dargestellt) wird auf das Sperrelement 4 eine Kraft in Richtung des Pfeils 70 ausgeübt. Diese Kraft drückt das Sperrelement 4 in die Sperrstellung 30. Wegen des Winkels 20 zwischen der Anlagefläche 8 des Oberteils 2 und der Anlagefläche 9 des Unterteils 3 wird das Sperrelement 4 durch die Kraft 70 zwischen den beiden Anlageflächen 8 und 9 verspannt. Dadurch, dass die beiden Anlageflächen 8 und 9 nicht parallel, sondern leicht geneigt zueinander angeordnet sind, kann sichergestellt werden, dass selbst im Falle von Verschleiß an dem Sperrelement 4 oder an einer der Anlageflächen 8 oder 9 das Sperrelement 4 in der Sperrstellung 30 stets spielfrei zwischen Oberteil 2 und Unterteil 3 verspannt ist. Verringert sich nämlich beispielsweise durch Verschleiß der Durchmesser des Sperrelements 4, kann das Sperrelement 4 durch die Kraft 70 ein Stück weiter nach unten gedrückt werden. Dies führt dann lediglich dazu, dass sich die Sperrstellung 30 leicht nach unten verlagert, wobei das Sperrelement 4 in der Sperrstellung 30 jedoch weiterhin spielfrei bleibt. Um das Sperrelement 4 sicher zwischen den Anlageflächen 8 und 9 verspannen zu können, beträgt der Winkel 20 erfindungsgemäß weniger als 15 Grad, vorzugsweise zwischen 3 Grad und 8 Grad, besonders bevorzugt etwa 5 Grad.

**Bezugszeichenliste:**

| | |
|---|---|
| 1 | Prothesengelenk |
| 2 | Oberteil |
| 3 | Unterteil |
| 4 | Sperrelement |
| 5 | Sperrhebel |
| 6 | Betätigungsmittel |
| 7 | Aussparung |
| 8 | Anlagefläche des Oberteils 2 |
| 9 | Anlagefläche des Unterteils 3 |
| 10 | Elastisches Element |
| 11 | Bremsschelle |
| 12 | Bremsachse |
| 13 | Steuerachse |
| 14 | Drehachse des Sperrhebels 5 |
| 15 | Pyramide |
| 16 | Spalt |
| 20 | Winkel zwischen Anlagefläche 8 des Oberteils 2 und Anlagefläche 9 des Unterteils 3 |
| 30 | Sperrstellung |
| 40 | Freigabestellung |
| 50 | Drehrichtung des Oberteils 2 gegenüber dem Unterteil 3 beim Beugen des Prothesengelenks 1 |
| 60 | Zugrichtung am Betätigungsmittel 6 zum Entriegeln des Prothesengelenks 1 |
| 70 | Richtung der vom elastischen Element 10 auf das Sperrelement 4 aufgebrachten Kraft |

## Patentansprüche

1. Prothesengelenk (1) mit Sperrvorrichtung, umfassend ein Oberteil (2), ein Unterteil (3) und ein Sperrelement (4),
wobei das Oberteil (2) und das Unterteil (3) so angeordnet sind, dass das Prothesengelenk (1) durch Rotieren des Oberteils (2) gegenüber dem Unterteil (3) beugbar ist, und
wobei das Sperrelement (4) so zwischen einer Freigabestellung (40) und einer Sperrstellung (30) überführbar ausgebildet ist, dass
in der Sperrstellung (30) das Prothesengelenk (1) in einem vorgegebenen Beugungszustand fixierbar ist durch Verspannung des Sperrelements (4) zwischen dem Oberteil (2) und dem Unterteil (3), und
eine Fixierung des Prothesengelenks (1) durch Überführen des Sperrelements (4) in die Freigabestellung (40) aufhebbar ist.

2. Prothesengelenk (1) mit Sperrvorrichtung nach Anspruch 1, weiterhin umfassend einen Sperrhebel (5), der so angeordnet und ausgebildet ist, dass das Sperrelement (4) durch Verlagern des Sperrhebels (5) zwischen der Freigabestellung (40) und der Sperrstellung (30) überführbar ist.

3. Prothesengelenk (1) mit Sperrvorrichtung nach Anspruch 2, weiterhin umfassend ein Betätigungsmittel (6), über das der Sperrhebel (5) verlagerbar ist.

4. Prothesengelenk (1) mit Sperrvorrichtung nach einem der vorhergehenden Ansprüche, wobei das Sperrelement (4) als Sperrbolzen ausgebildet ist.

5. Prothesengelenk (1) mit Sperrvorrichtung nach einem der vorhergehenden Ansprüche, wobei das Sperrelement (4) im Oberteil (2) gelagert ist.

6. Prothesengelenk (1) mit Sperrvorrichtung nach Anspruch 5, wobei das Oberteil (2) eine Aussparung (7) aufweist, in der das Sperrelement (4) verschiebbar gelagert ist.

7. Prothesengelenk (1) mit Sperrvorrichtung nach einem der vorhergehenden Ansprüche, wobei das Sperrelement (4) in der Sperrstellung (30) an einer Anlagefläche (8) des Oberteils (2) und an einer Anlagefläche (9) des Unterteils (3) anliegt, wenn das Prothesengelenk (1) fixiert ist, und die Anlagefläche (8) des Oberteils (2) zu der Anlagefläche (9) des Unterteils (3) in einem Winkel (20) zwischen 3° und 8°, vorzugsweise etwa 5°, angeordnet ist.

8. Prothesengelenk (1) mit Sperrvorrichtung nach einem der vorhergehenden Ansprüche, weiterhin umfassend ein elastisches Element (10), das eine Kraft erzeugt, die bestrebt ist, das Sperrelement (4) in die Sperrstellung (30) zu überführen.

9. Prothesengelenk (1) mit Sperrvorrichtung nach einem der vorhergehenden Ansprüche, weiterhin umfassend eine Bremsschelle (11), die so zwischen dem Oberteil (2) und dem Unterteil (3) angeordnet ist, dass die Bremsschelle (11) gegenüber dem Oberteil (2) um eine Bremsachse (12) drehbar ist und gegenüber dem Unterteil (3) um eine Steuerachse (13) drehbar ist, wobei in der Sperrstellung (30) des Sperrelements (4) die Drehbarkeit des Oberteils (2) gegenüber der Bremsschelle (11) um die Bremsachse (12) sperrbar ist.

10. Prothesengelenk (1) mit Sperrvorrichtung nach Anspruch 9, wobei in der Sperrstellung (30) des Sperrelements (4) auch die Drehbarkeit der Bremsschelle (11) gegenüber dem Unterteil (3) um die Steuerachse (13) sperrbar ist.
